# EUROPEAN PATENT APPLICATION

(11) **EP 4 397 969 A1**
(43) Date of publication of application: **10.07.2024**
(21) Application number: 23150620.5
(22) Date of filing: 06.01.2023
(51) Int. Cl.: G01N 24/08, G01R 33/465, G01R 33/48

(54) **IMPROVEMENTS IN AND RELATING TO MEASUREMENT OF SOLUTIONS USING NUCLEAR MAGNETIC RESONANCE**

(71) Applicant: University of Limerick, Limerick V94 T9PX (IE)
(72) Inventor: O'REILLY, Emmet, Limerick, V94 T9PX (IE); O'REILLY, John, Limerick, V94 T9PX (IE)
(74) Representative: Appleyard Lees IP LLP

(57) **Abstract**

According to the present disclosure there is provided a method comprising providing a solution comprising a plurality of solution components; selecting a reference component from among the solution components; and using nuclear magnetic resonance, NMR, to measure a parameter of the reference component. The present disclosure also provides an apparatus comprising a NMR unit and a processor configured to perform the method.

## Description

### FIELD

The present disclosure generally relates to process analytical technology (PAT). The present disclosure relates to a method, in particular a method of measurement of a solution. The present disclosure also relates to an apparatus configured to perform the method.

### BACKGROUND TO THE INVENTION

Buffer solutions are used in the manufacture of biopharmaceuticals. A typical biopharmaceutical manufacturing plant can use thousands of litres of buffer per day. Manufacturing buffers with the required Critical Quality Attributes (CQAs) is an essential process in biotherapeutic manufacture. Traditional buffer manufacture requires significant floor space and resources. Therefore, in an effort to alleviate this burden, many facilities are moving towards in-line conditions systems that generate buffer solutions as required via dilution of stocks. However traditional buffer manufacturing remains commonplace.

Current techniques for verifying the composition, concentration and pH of buffer solutions are outdated, time consuming and involve techniques that require extensive calibration to ensure they are operating correctly or suffer from significant limitations when implemented in an "at-line" or "in-line" configuration. The also lack compatibility with respect to automation or continuous manufacturing. Given the high monetary value of biotherapeutic products, significant time and effort is invested in establishing buffer CQAs. When performed with equipment that requires extensive calibration or is only suitable for off-line analysis, this leads to significant bottlenecks at key points in the manufacturing process. Two examples of this are during Virus Inactivation (VIN) or the verification of buffers produced by in-line conditioning (IC) or in-line buffer dilution (IBD).

Virus inactivation is a key step in biotherapeutic manufacture and is mandated by regulatory agencies such as the Food and Drugs Administration (FDA) and the European Medicines Agency (EMA). This step involves reducing the pH of the product solution from approximately pH 5.6 to approximately pH 3.6 for a period of time followed by increasing the pH to approximately pH 5.6 once more. Prolonged exposure of the biotherapeutic product to low pH values can lead to product damage and loss of therapeutic effectiveness. Currently this process requires significant off-line analysis and pH titrations to establish the required acid/base concentrations for pH adjustment. Sterilise in Place (SIP) and Clean in Place (CIP) requirements significantly impact the use of in-line or at line pH measurements. The lack of reliable, efficient and robust at-line/in-line measurement tools adds significant time to downstream production processes such as VIN resulting in manufacturing bottlenecks.

There is currently no real-time at line method for verification of the concentration and compositions of buffer solutions produced by in-line conditioning (IC) or Inline buffer dilution (IBD), or other buffer intensive processes currently being adopted in industry. Commercially available in-line conditioning methods verify the composition and concentration of manufactured buffer solutions via a combination of conductivity measurements and mass flow metering. These methods do not measure actual concentration or composition; instead, data from these methods is used to "infer" final buffer concentration. As a result, manufacturers are required to verify actual buffer composition and concentration for regulatory and operational purposes resulting in the requirement for secondary analytical analysis. Furthermore, there are concerns regarding the use of buffers with inferred properties in the manufacture of high-value biopharmaceutical products, thus requiring verification of buffer properties using analytical instrumentation before use, resulting in production delays.

Low field NMR can be used for both qualitative and quantitative analysis. It has not been applied extensively for pH determination. Existing pH determination using low field NMR requires the addition of a dedicated "reference" component to the sample being analysed and the use of deuterated H₂O. The dedicated reference component is added to the sample to reference the shifting of nuclear magnetic resonance (NMR) peaks as a function of pH. The addition of deuterated H₂O or any other dedicated reference component in a biopharmaceutical manufacturing process is strictly forbidden as the buffer solution and therapeutic product will be contaminated.

Furthermore, in existing approaches, during verification of the composition concentration or pH of a manufactured buffer solution physical contact must be made between instrumentation (such as a probe) and the manufactured buffer solution (or a sample thereof). There is thus a risk to sterility of the solution. This risk increases with consecutive sampling which may be required to monitor buffer CQAs over a period of time.

### SUMMARY OF THE INVENTION

It is one aim of the present invention, amongst others, to provide an improved system and/or method thereof and/or address one or more of the problems discussed above, or discussed elsewhere, or to at least provide an alternative system and/or method.

A first aspect provides a method comprising: providing a solution comprising a plurality of solution components; selecting a reference component from among the solution components; using nuclear magnetic resonance, NMR, to measure a parameter of the reference component.

In one example, the method further comprises: tuning operation of an NMR unit; using NMR to re-measure the parameter of the reference component.

In one example, the method further comprises: using the measured parameter of the reference component as a calibration; and based on the calibration, using NMR to measure a parameter of the solution.

In one example, the method further comprises: measuring the parameter of the solution by measuring a shift of the NMR peak of one or more other solution components that are not the reference component with respect to the calibration.

In one example, the parameter of the reference component and/or the parameter of the solution is one or more of: liquid composition; concentration; flow rate; pH; osmolality; conductivity; mixing efficiency; and dissolved oxygen content.

In one example, the parameter of the reference component and/or the parameter of the solution is pH, and the pH is determined from one or more parameters of the NMR signal peak, optionally peak position, peak shape at a given temperature, area under the peak and/or peak height.

In one example, the method is performed as part of a biopharmaceutical manufacturing process.

In one example, the method is performed without addition of a dedicated reference component to the solution.

In one example, a sample is removed from a tank for measurement and returned to the tank after measurement.

In one example, the method is performed without measuring a parameter of an added dedicated reference component and/or without using a measured parameter of an added dedicated reference component as a calibration.

In one example, the solution comprises one or more of, as solution components: a buffer; a protein solution; a chromatography solution including gradients; an excipient solution; an additive solution; a detergent solution; a weak acid; an organic solution; a salt solution; a base.

In one example, the buffer is one or more of: sodium acetate; acetic acid; citric acid; Tris HCL; Tris base; potassium acetate; sodium chloride; disodium phosphate; monosodium phosphate.

In one example, the solution comprises a buffer, and the association-dissociation equilibrium state of the buffer is determined from one or more parameters of the NMR signal peak, optionally peak position, peak shape, area under the peak and/or peak height.

In one example, the solution is flowing/dynamic or static.

In one example, the method further comprises: adding a solution component during a virus inactivation, VIN, process; and selecting the added solution component as the reference component from among the solution components.

In one example, the reference component is water, optionally water at 4.79ppm. In one example, the reference component is water observed at 4.79ppm in a ¹H NMR spectrum at a frequency of 60MHz.

In one example, the method is performed during in-line conditioning, IC, and/or in-line buffer dilution, IBD. In one example, the method is performed during virus inactivation (VIN).

In one example, NMR signals are sensed by a sensor without physical contact of the sensor with the solution. In this way, the sterility of the solution is not compromised.

A second aspect provides an apparatus comprising a NMR unit and a processor configured to perform a method according to the first aspect.

In one example, the apparatus is configured to receive a solution from an IC, IBD system, or VIN/Buffer exchange stage in a biopharmaceutical manufacturing process. The reference component from among the solution components may be selected by the processor, or may be selected by a user and provided as input to the processor. The processor may control the NMR unit to measure a parameter of the reference component, preferably measure pH of the reference component. The processor may control the NMR unit to measure a parameter of the reference component and one or more other solution components from which it can calculate a parameter of the solution, preferably pH

It will of course be appreciated that features described in relation to one aspect of the present invention may be incorporated into other aspects of the present invention. For example, the apparatus of the second aspect of the invention may incorporate any of the features described with reference to the method of the first aspect of the invention and vice versa.

Other preferred and advantageous features of the invention will be apparent from the following description.

### BRIEF DESCRIPTION OF THE FIGURES

For a better understanding of the invention, and to show how embodiments of the same may be carried into effect, reference will now be made, by way of example only, to the accompanying diagrammatic drawings in which:
Figure 1 shows a schematic method;
Figure 2 shows an NMR spectrum changing as a function of pH;
Figure 3 shows a prior art process;
Figure 4 shows a system;
Figure 5 shows in-line pH measurement during a VIN process;
Figure 6 shows in-line buffer verification;
Figure 7 shows an apparatus;
Figure 8 shows in-line pH measurement during a VIN process;
Figure 9 shows in-line buffer verification for an in-line conditioning system; and
Figure 10 shows an apparatus.

### DETAILED DESCRIPTION OF THE INVENTION

According to the present invention there is provided a method and apparatus as set forth in the appended claims. Other features of the invention will be apparent from the dependent claims, and the description which follows.

In overview, the present disclosure relates to a low-field NMR method for providing contactless scientific measurements in relation to solutions used in pharmaceutical and Biopharmaceutical manufacturing including buffer solutions, aqueous solutions and organic solutions. In one example, a method for providing critical quality attributes CQAs (such as buffer pH, buffer composition, buffer concentration, acid/base concentration, flow rate, dissolved oxygen content, protein concentration, and mixing efficiency) measurements of buffer solutions as part of a Virus Inactivation (VIN) step, buffer critical or buffer exchange process or as part of buffer manufacture in in an IB/ILC process. The method permits determination of pH without the requirement for additional reference components to be added to the solution being measured whereby pH is determined based on peak position and/or peak shape with respect to the peak position of another component of the buffer. The method employs components of the sample being measured as a reference for the measurement. The method employs components of the sample being measured as a calibrating standard. In an example, the method provides offline, at-line or inline measurements as part of a buffer critical biopharma manufacturing process. In an at line, in line, or offline configuration the method and/or apparatus may replace currently used titrimetric methods and devices used in the lab for pH determination and acid/base calculations. In an at-line configuration the present apparatus may be deployed adjacent to VIN/buffer exchange tanks. Sample is placed in the device for measurement using existing glass or plastic SUS tubing. In an example, a sample enters and leaves the device in an at-line, stop-start configuration prior to being moved to waste. In an example the sample is analysed at-line in a stop-start configuration and returned to the tank. If deployed in this format the method and/or apparatus removes the requirement for weighing measurements and titrimetric assays as part of virus inactivation. When deployed as part of Virus Inactivation (VIN) and buffer exchange processes the method and/or apparatus provides the amount of acid/base required to reach the target pH.

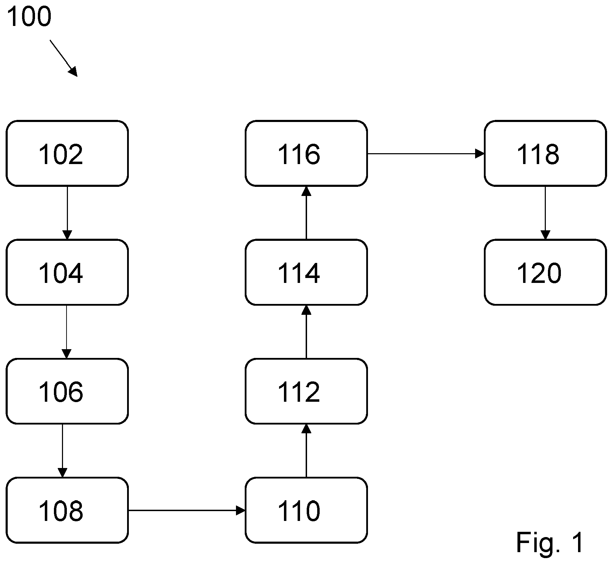

Referring to Figure 1, a method 100 is shown according to a first aspect of the invention.

Step 102 comprises providing a solution comprising a plurality of solution components. Step 104 comprises selecting a reference component from among the solution components. Step 106 comprises using nuclear magnetic resonance (NMR) to measure a parameter of the reference component.

Selecting a reference component from among the solution components means that of the components present in the solution there is a reference component, a parameter of which is to be measured. This is in contrast to existing approaches in which one or more "dedicated" reference components are purposefully added to a sample of the solution to provide the reference component. Instead, in the present case, a component of the solution (e.g., the existing, or original solution) is selected to be the reference component. In this way, it is not necessary to add an additional component to the solution solely for use as a reference component. Furthermore, in the present case, where a sample is extracted for measurement, the sample may be reintroduced to the bulk as an additional reference component is not introduced which would be undesired in the bulk.

Step 106 may comprise using low-field NMR to measure a parameter of the reference component. Low-field NMR is a term well understood by those skilled in the art. In a broad sense, low-field NMR is NMR that is not conducted in superconducting high-field magnets. Low-field NMR may refer to the use of magnetic fields based on permanent magnets of the order of 1 Tesla or less and operating at a frequency between 10MHz and 100MHz. In the context of the present invention, low-field NMR units (i.e., the instruments themselves) are relatively small, compared with high-field NMR units, thus making them portable (whereby the same instrument may be employed in process development or on the production floor) and also suitable for bench-top applications.

The method 100 may further comprise Steps 108 and 110. Step 108 comprises tuning operation of an NMR unit. Step 110 comprises using NMR to re-measure the parameter of the reference component.

Tuning operation of the NMR unit follows measuring a parameter of the reference component. Specifically, where the NMR spectrum (generated using NMR to measure the parameter of the reference component) contains peaks that have a large width, the NMR unit may be tuned to reduce the peak width, thereby increasing the accuracy of peak position measurements. Tuning operation may refer to adjusting the operation of the NMR unit. Tuning may also form part of the method.

NMR may then be used to re-measure the parameter of the reference component. In this way, the peak position is accurately defined.

In this way, the parameter of the reference component may then be used as an internal calibration.

The method 100 may further comprise Steps 112 and 114. Step 112 comprises using the measured parameter of the reference component as a calibration. Step 114 comprises using NMR to measure a parameter of the solution, based on the calibration.

By using the measured parameter of the reference component as a calibration, parameters of the solution can be accurately measured. Furthermore, in this way, the concentration of the solution (e.g., a buffer solution) can be determined by referencing the buffer NMR peak to that of the water NMR peak, without the need for calibrating standards.

The method 100 may further comprise Step 116. Step 116 comprises measuring the parameter of the solution by measuring a shift of the NMR peak of one or more other solution components that are not the reference component with respect to the calibration. That is, the shift may be measured from the calibration.

In a specific example, water is selected as the reference component from among the solution components, and observed at 4.79ppm in a ¹H NMR spectrum at a frequency of 60MHz. It will be appreciated by those skilled in the art that "ppm" does not refer to concentration, but rather peak position. By using this peak of water as a calibration, the shift from the water peak of spectral peaks corresponding to other components, in combination with the peak height and peak shape, can be used to determine the pH of the solution. It will be appreciated by those skilled in the art that the shift is a proton (¹H) chemical shift, and may be referred to as "water, having a ¹H chemical shift of 4.79 ppm" or "water, giving a characteristic signal at 4.79ppm in a ¹H NMR spectrum".

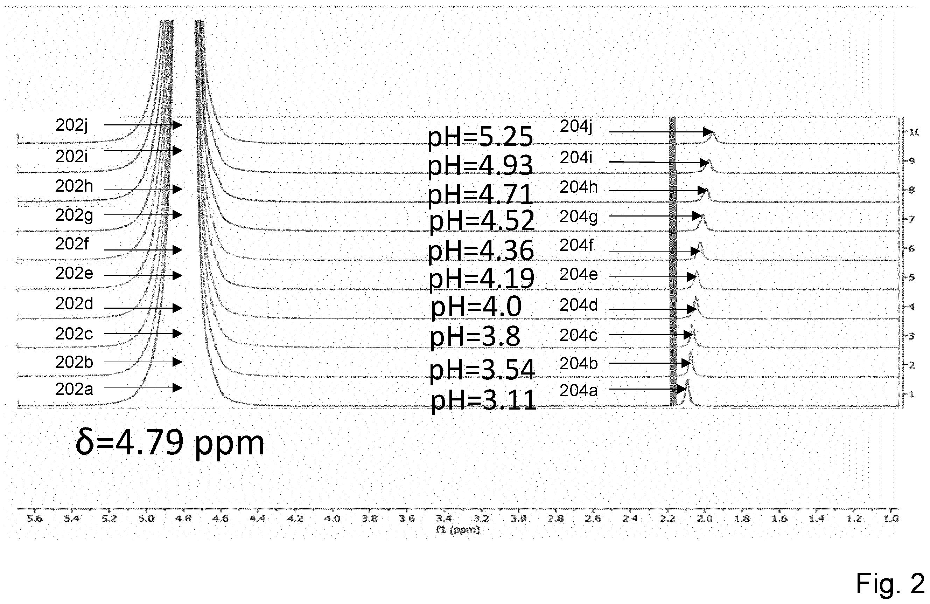

For example, referring to Figure 2, an NMR spectrum is shown. The spectral peak of the reference component is indicated at 202a - 202j. The reference component is water. This peak is used as a calibration, and spectral peaks corresponding to other components are indicated at 204a - 204j. The shift from the water peak, in combination with peak height and peak shape, can be used to determine the pH of the other solution components. At maximum and minimum pH values the peak will no longer shift with respect to 4.79ppm however the peak shape will continue to change. The NMR spectrum may be used to measure a parameter of the reference component and one or more other solution components from which it can calculate a parameter of the solution, preferably pH.

Referring back to Figure 1, in an example, the parameter of the reference component and/or the parameter of the solution may be one or more of: liquid composition; concentration; flow rate; pH; osmolality; conductivity; mixing efficiency; protein concentration; and dissolved oxygen content.

These parameters may be referred to as "Critical Quality Attributes (CQAs)".

In an example, the parameter of the reference component and/or the parameter of the solution is pH, and the pH is determined from one of more parameters of the NMR signal peak. In an example, the one or more parameters of the NMR signal peak includes peak position, peak shape at a given temperature, and/or peak height.

In an example, the method 100 is performed as part of a biopharmaceutical manufacturing process. The method 100 may be a method for use in biopharmaceutical manufacturing.

The method 100 is particularly advantageous when performed as part of a biopharmaceutical manufacturing process as real-time verification of parameters (including CQAs such as concentration and composition) can be performed. Moreover, such parameters can be measured without inference based on data.

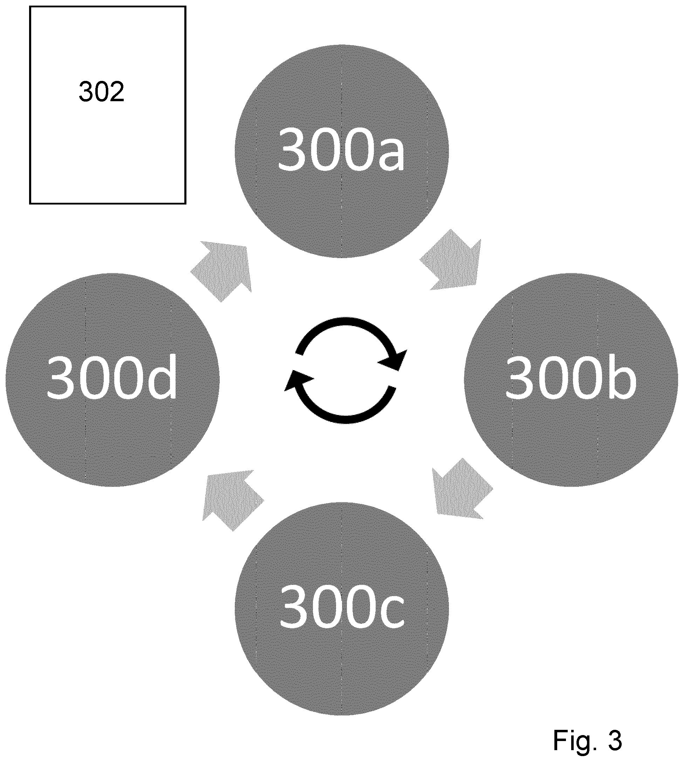

Referring to Figure 3, a biopharmaceutical manufacturing process according to the prior art is shown. Step 300a comprises decanting a sample to a sampling bag from a VIN reactor 302. Step 300b comprises performing off-line pH measurement of the sample. Step 300c comprises scaleup calculation. Step 300d comprises pH adjustment of the solution in the VIN reactor 302. The process returns to Step 300a. It is notable that the prior art process involves time consuming off-line measurement, the performance of "point in time" (i.e., instantaneous) measurement, requires pH meter calibration, provides limited process understanding and has a low level of automation.

Referring back to Figure 1, in an example, the method is performed without addition of a dedicated reference component to the solution.

In this way, risk of contamination of the buffer solution is mitigated and, in the context of buffer solution manufacture and biopharmaceutical manufacture, the manufacturing process is simplified.

In an example, a sample of the solution is removed from a tank for measurement, and the sample is returned to the tank after measurement.

This is not possible using commercially available methods which require addition of a dedicated reference component to the solution which renders the sample unusable for reintroduction to the bulk in addition to sterility being compromised.

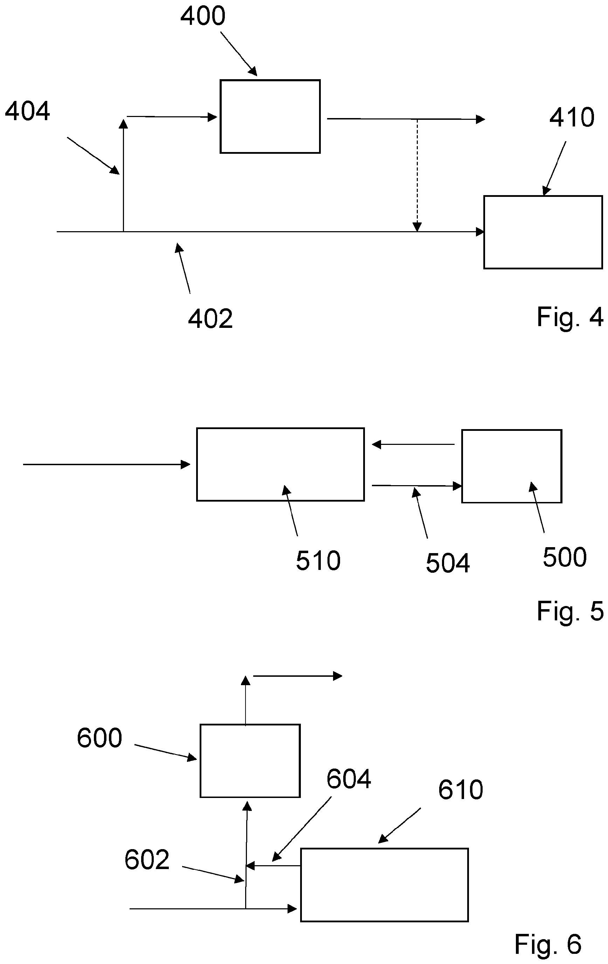

In an example, a flow channel may divert a sample of the solution from an in-line conditioning (IC) or in-line buffer dilution (IBD) system. The sample may be diverted away from the main flow channel via a sample flow channel. The sample may be measured at, or in, the sample flow channel. Following measurement, the sample may be disposed of as waste, or may be returned to the bulk by merging with the main flow channel. Such a method allows real time sampling of IC and IBD system performance, allows performance of the IC and IBD system to be monitored in the time domain, and allows for incorporation of feedback loops if enhanced automation is desired. An example of such a process is shown in Figure 4, in which the output of an IC or IBD system is measured. The sample is diverted away from the main flow channel 402 via sample flow channel 404. The sample is measured by apparatus 400 in the sample flow channel 404. Following measurement, the sample may be disposed of as waste, or may be returned to the bulk by merging with the main flow channel 402. In this example, the main flow channel 402 may channel the output of the IC/IBD system to a reservoir 410 for storage. Whilst the channelling of the output to a reservoir downstream of the measurement of the solution may inhibit the validation of final parameters of the output, feedback loops can be incorporated to adjust the output appropriately.

In an example, the sample may be removed from a reservoir, the sample measured, and the sample disposed of as waste or returned to the reservoir. Such a method is relatively straightforward to implement compared with other approaches, allows active monitoring of overall solution concentrations in the time domain, and allows analysis of overall solutions irrespective of the individual components delivered. An example of such a process is shown in Figure 5. The sample is removed from a reservoir or tank 510 via sample flow channel 504 and measured by apparatus 500 in the sample flow channel. Following measurement, the sample may be disposed of as waste, or may be returned to the reservoir or IC/IBD flow channel.

In an example, a flow channel may divert a part of a sample of the solution from an IC or IBD system, and a further part of the sample may be removed from a reservoir. The sample of the solution can then be measured and the sample disposed of as waste or merged with the IC or IBD main flow channel. This allows real time sampling of IC and IBD system performance, allows performance of the IC and IBD system to be monitored in the time domain, allows for incorporation of feedback loops if desired, and also provides validation for final solution parameters or properties. An example of such a process is shown in Figure 6. Here, a part of a sample of the solution is diverted from the output of an IC or IBD system via sample flow channel 602 and a further part of the sample is removed from a reservoir 610 via sample flow channel 604. The sample parts are combined and measured at apparatus 600. Following measurement, the sample may be disposed of as waste, or may be returned to the reservoir or IC/IBD flow channel.

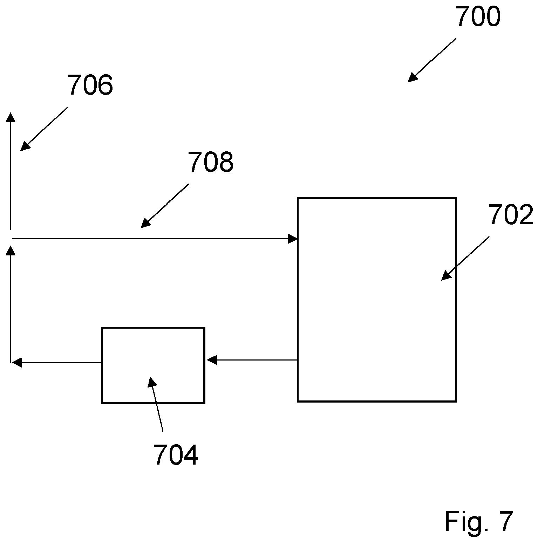

Referring to Figure 7, an exemplary system 700 is shown. The system 700 may be reflective of a VIN process, and may also exemplify the benefits of the present method and apparatus to an existing VIN process. The system 700 is configured to facilitate removal of a sample from a reservoir 702, measure the sample using an apparatus 704 comprising an NMR unit and a processor configured to perform the method herein described, and the sample disposed of as waste by waste-line 706 or returned to the reservoir 702 by return-line 708. It is notable that the present solution provides for robust real-time analysis of pH, the knowledge of appropriate amounts of acid/base to add and allows monitoring of changes in concentration in real time. Furthermore, the present solution negates any need for Clean In Place (CIP) or Sterilise In Place (SIP) processes, provides for full online process control, enables enhanced automation, and is compatible (i.e., retrofittable) with existing sampling equipment.

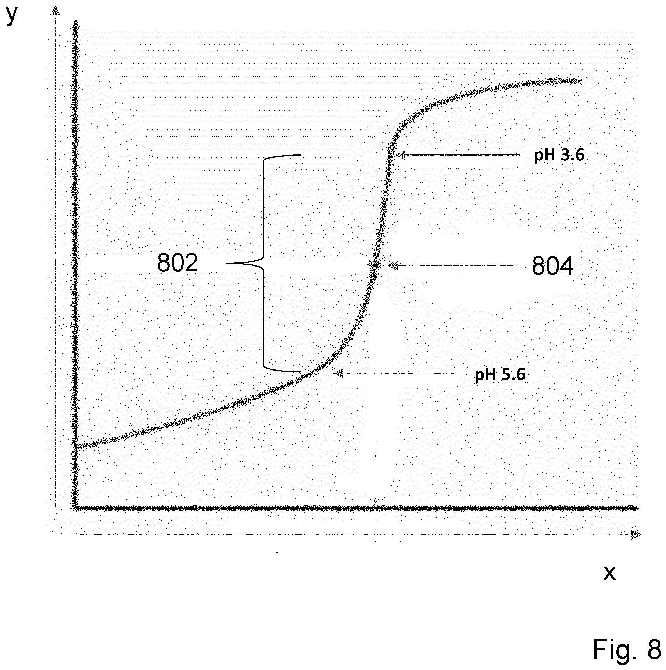

Referring to Figure 8, in-line pH measurement during a VIN process is shown. Acid or base is added to the solution depending on the stage of the process. pH is shown (x-axis) against volume of acid or base added to the solution (y-axis). The range 802 indicates the volume of acid or base required for the pH adjustment, with the maximum at 5.6 and minimum at 3.6. The current pH is indicated at 804. In existing VIN processes, the amount of acid/base required for a necessary pH adjustment is estimated based on off-line measurements. The present solution provides the current pH (measured based on peak position, shape and height) and the precise amount of acid/base required for pH adjustment in an at-line configuration. In some cases, lowering the pH below a certain threshold can result in damage to the product, therefore it is important that the pH can be accurately controlled. This is not possible with existing VIN processes, but is provided for by the present solution. The present solution also provided the essential real time data required for automation of the VIN process.

Referring back to Figure 1, in an example, the method is performed without removal of a sample of the solution. The method may be performed without addition of a dedicated reference component to the sample.

In an example, the method is performed without measuring a parameter of an added dedicated reference component and/or without using a measured parameter of an added dedicated reference component as a calibration.

In an example, the solution comprises one or more of, as solution components: a buffer; a protein solution; a chromatography solution including gradients; an excipient solution; an additive solution; a detergent solution; a weak acid; an organic solution; a salt solution; a base.

In an example, the buffer is one or more of: sodium acetate; acetic acid; citric acid; Tris HCL; Tris base; potassium acetate; sodium chloride; disodium phosphate; monosodium phosphate.

In an example, the solution comprises a buffer and the association/dissociation equilibrium state of the buffer is determined from one or more parameters of the NMR signal peak. In one example, the parameters of the NMR signal peak are: peak position, peak shape, area under the peak, and/or peak height.

The association-dissociation equilibrium state informs the pH measurement.

In an example, the solution is flowing or "dynamic". In another example, the solution is static. That is, in an example, a sample of the solution taken for measurement may be flowing, for example in the case where the sample is diverted by a flow channel from a main flow channel in an IC or IBD system. In another example, a sample of the solution taken for measurement may be static, for example a volume of the solution being extracted from the bulk and inserted into an apparatus configured to perform the method.

The method 100 may further comprise Step 118 and 120. Step 118 comprises adding a solution component during a virus inactivation, VIN, process. Step 120 comprises selecting the added solution component as the reference component from among the solution component. It will be apparent to the skilled person that any added component (e.g., a base or acid) is part of the manufacturing process, and not a dedicated species added only for reference.

The added solution component may be Tris HCL or Tris base. The added solution component may be added when parameter of the solution is within a predetermined range. The parameter of the solution may be pH. The predetermined range may be a pH range in which the peak position of the added solution component does not shift or move. In this way, the added solution component may be used as a reference component (i.e., an added reference component) during the VIN process.

In an example, the reference component is water. The water may have a peak position of 4.79ppm.

Advantageously, in this way, water in the sample may be used as the reference component. That is, the sample itself provides its own reference component. Furthermore, the peak position of water in the solution does not shift due to changes in pH, facilitating its use as a reference component. The water may be an existing majority component of the sample. That is, water may have the largest concentration in the sample relative to other components of the sample. The inventors have identified that other reference components comprised in the sample itself may be used as the reference component, providing the shift due to changes in pH is negligible or is predictable.

In an example, the method is performed during a Virus inactivation (VIN) or in-line conditioning (IC) or in-line buffer dilution (IBD).

Such a method is highly advantageous as real-time verification of buffer concentration and composition (without inference from secondary sources) can be achieved during an IC or IBD process, thereby meeting regulatory requirements and ensuring quality of high value biopharmaceutical products.

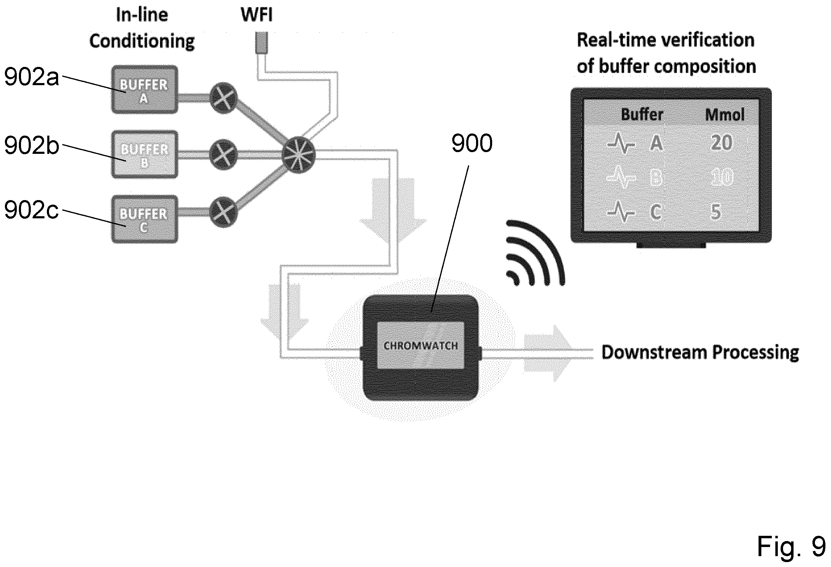

Referring to Figure 9, in-line buffer verification for an in-line conditioning system is shown. In this example, water for injection (WFI) encounters an in-line conditioning system comprising a plurality of buffer solutions 902a - 902c. An apparatus 900 comprising an NMR unit and a processor configured to perform the method herein described performs real-time verification of the buffer composition. The apparatus 900 may comprise a communication unit configured to communicate the verification result to a remote device, such as a PC, for display to a user. The solution continues to downstream processing, without risk of contamination or risk to sterility.

Referring back to Figure 1, in an example, NMR signals are sensed by a sensor without physical contact of the sensor with the solution.

This may otherwise be referred to as "contactless" measurement. Whilst prior art system require physical contact of a sensor probe with a solution (or a sample thereof), the present method facilitates measurement without such physical contact. The risk of contamination of the solution is thereby mitigated, and any sample may be reintroduced to the bulk thereby reducing waste. As outlined above the method herein removes the requirement for clean in place (CIP) or sterilise in place (SIP) procedures.

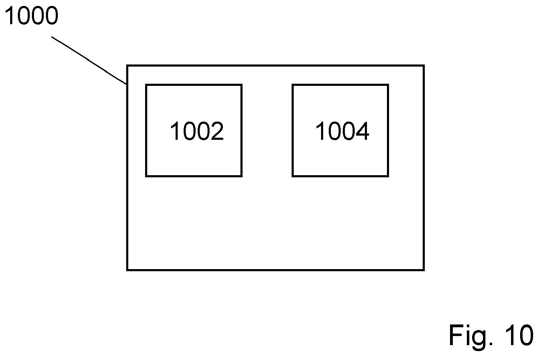

Referring to Figure 10, an apparatus 1000 is shown according to a second aspect of the invention. The apparatus 1000 comprises an NMR unit 1002 and a processor 1004 configured to perform the method 100 herein described. The apparatus 1000 may comprise any or all features of any apparatus herein described. The apparatus 1000 may be for use VIN, buffer exchange or in an in-line conditioning system and/or an in-line buffer dilution system. The apparatus 1000 may be incorporated in such systems. That is, such systems may comprise the apparatus 1000.

NMR units are well known in the art. The NMR unit may otherwise be referred to as an NMR instrument. An NMR unit comprises a number of components, including one or more magnetic coils, one or more permanent magnets, a sweep generator, a radio frequency (RF) transmitter, an RF detector, a recorder and a readout system.

It will be appreciated that any features of the method according to the first aspect of the invention may be incorporated into the apparatus according to the second aspect of the invention. In particular, the processor may be configured to perform any of the method steps of method 100 according to the first aspect of the invention. In particular, the apparatus 1000 may comprise an NMR unit 1002 and a processor 1004 configured to: from a solution comprising a plurality of solution components, select or receive a selection of a reference component from among the solution components; and control the NMR unit 1002 to measure a parameter of the reference component using NMR.

The apparatus 1000 may receive a solution from an IC or IBD system. The reference component from among the solution components may be selected by the processor 1004, or may be selected by a user and provided as input to the processor 1004. The processor 1004 may control the NMR unit 1002 to measure a parameter of the reference component, preferably measure pH of the reference component.

### Definitions

Although a preferred embodiment has been shown and described, it will be appreciated by those skilled in the art that various changes and modifications might be made without departing from the scope of the invention, as defined in the appended claims and as described above.

At least some of the example embodiments described herein may be constructed, partially or wholly, using dedicated special-purpose hardware. Terms such as 'component', 'module' or 'unit' used herein may include, but are not limited to, a hardware device, such as circuitry in the form of discrete or integrated components, a Field Programmable Gate Array (FPGA) or Application Specific Integrated Circuit (ASIC), which performs certain tasks or provides the associated functionality. In some embodiments, the described elements may be configured to reside on a tangible, persistent, addressable storage medium and may be configured to execute on one or more processors. These functional elements may in some embodiments include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. Although the example embodiments have been described with reference to the components, modules and units discussed herein, such functional elements may be combined into fewer elements or separated into additional elements. Various combinations of optional features have been described herein, and it will be appreciated that described features may be combined in any suitable combination. In particular, the features of any one example embodiment may be combined with features of any other embodiment, as appropriate, except where such combinations are mutually exclusive. Throughout this specification, the term "comprising" or "comprises" means including the component(s) specified but not to the exclusion of the presence of others.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. A method comprising:
providing a solution comprising a plurality of solution components;
selecting a reference component from among the solution components;
using nuclear magnetic resonance, NMR, to measure a parameter of the reference component.

2. The method according to claim 1, further comprising:
tuning operation of an NMR unit;
using NMR to re-measure the parameter of the reference component.

3. The method according to claim 1 or 2, further comprising:
using the measured parameter of the reference component as a calibration; and
based on the calibration, using NMR to measure a parameter of the solution.

4. The method according to any one of the preceding claims, further comprising:
measuring the parameter of the solution by measuring a shift of the NMR peak of one or more other solution components that are not the reference component with respect to the calibration.

5. The method according to any one of the preceding claims, wherein the parameter of the reference component and/or the parameter of the solution is one or more of: liquid composition; concentration; flow rate; pH; osmolality; conductivity; mixing efficiency; and dissolved oxygen content.

6. The method according to claim 5, wherein the parameter of the reference component and/or the parameter of the solution is pH, and the pH is determined from one or more parameters of the NMR signal peak, optionally peak position, peak shape at a given temperature, area under the peak, and/or peak height.

7. The method according to any one of the preceding claims, wherein the method is performed as part of a biopharmaceutical manufacturing process.

8. The method according to any one of the preceding claims, wherein a sample is removed from a tank for measurement and returned to the tank after measurement.

9. The method according to any one of the preceding claims, wherein the method is performed without measuring a parameter of an added dedicated reference component and/or without using a measured parameter of an added dedicated reference component as a calibration.

10. The method according to any one of the preceding claims, wherein the solution comprises one or more of, as solution components: a buffer; a protein solution; a chromatography solution including gradients; an excipient solution; an additive solution; a detergent solution; a weak acid; an organic solution; a salt solution; a base.

11. The method according to any one of the preceding claims, further comprising:
adding a solution component during a virus inactivation, VIN, process; and
selecting the added solution component as the reference component from among the solution components.

12. The method according to any one of the preceding claims, wherein the reference component is water, optionally water at 4.79ppm in a ¹H NMR spectrum at a frequency of 60MHz.

13. The method according to any one of the preceding claims, wherein the method is performed during in-line conditioning, IC, and/or in-line buffer dilution, IBD.

14. The method according to any one of the preceding claims, wherein NMR signals are sensed by a sensor without physical contact of the sensor with the solution.

15. An apparatus comprising a NMR unit and a processor configured to perform the method of any preceding claim.
